# EUROPEAN PATENT SPECIFICATION

(11) **EP 2 922 480 B1**
(45) Date of publication and mention of the grant of the patent: **30.12.2020**
(21) Application number: 13856790.4
(22) Date of filing: 20.11.2013
(51) Int. Cl.: A61B 17/06, A61M 5/00, A61M 5/32, A61M 25/00, A61M 25/01, A61M 25/02

(54) **CATHETER IN BAG WITHOUT ADDITIONAL PACKAGING**
KATHETER IN EINEM BEUTEL OHNE ZUSÄTZLICHE VERPACKUNG
CATHÉTER DANS UN SAC SANS EMBALLAGE SUPPLÉMENTAIRE

(30) Priority: 20.11.2012 US 201213682420
(43) Date of publication of application: 30.09.2015
(60) Divisional application: 20198654.4
(73) Proprietor: C.R. Bard, Inc., Murray Hill, NJ 07974 (US)
(72) Inventor: CONWAY, Anthony, J., Chatfield, MN 55923 (US); GRINDE, Sarah, L., Spring Grove, MN 55974 (US)
(74) Representative: Hoffmann Eitle
(86) International application number: PCT/US2013/071060
(87) International publication number: WO 2014/081859

(56) References cited:
- EP-A1- 1 115 450
- EP-A1- 2 072 075
- WO-A1-2012/134804
- WO-A2-03/008028
- US-A- 5 402 886
- US-A1- 2006 025 753
- US-A1- 2007 161 971
- US-A1- 2009 208 368
- US-B2- 6 602 244

## Description

### Field of the Invention

The present invention relates to a urinary catheter and collection bag that can be provided without additional packaging.

### Background of the Invention

Urinary catheter kits include a urinary catheter, a urine collection bag, and, for example, a packet including an antiseptic wipe, a pair of gloves, an absorbent pad, a packet containing water, a lubricant, instructions or product information, or several of these items. Such kits are provided in a package to keep the catheter and other components clean or sterile and to keep them together. For example, the kit can be provided in a bag or tray. The user or health care professional opens the package, removes the several components, and inserts the catheter into the subject. There remains a need for a convenient system to provide clean components and a sterile catheter.

EP 2 072 075 A1 discloses a catheter assembly with a folded urine collection bag.

US 2006/0025753 A1 discloses a urinary catheter assembly.

WO 03/008028 A2 discloses a lubricating and gripping device for urinary catheter package.

WO 00/16843 discloses a catheter set.

US 2009/0208368 A1 discloses a urinary catheter, catheter packaging assembly and method of use.

### Summary of the Invention

The present invention relates to a urinary catheter and collection bag that can be provided without additional packaging. The present invention includes a urinary catheter set. This set includes a collection bag and a urinary catheter. The catheter is inside the collection bag. The collection bag is configured as a package for the set. And, the collection bag is the only package for the set.

In an embodiment, the present invention includes a case of urinary catheter sets. The case includes a container and a plurality of the present catheter sets. The plurality of sets in in the container. The collection bag is the only package for the set.

### Brief Description of the Figures

Figure 1 schematically illustrates an embodiment of the present collection bag and urinary catheter.
Figure 2 schematically illustrates a cross sectional view of an embodiment of a bag according to the present invention in a configuration in which it has been wrapped or folded to form a cavity.
Figure 3 schematically illustrates a cross sectional view of another embodiment of a bag according to the present invention in a configuration in which it has been wrapped or folded to form a cavity.
Figure 4 schematically illustrates a cross sectional view of an embodiment of a bag with extensions according to the present invention in a configuration in which it has been wrapped or folded to form a cavity.

### Detailed Description of the Invention

The present invention relates to a urinary catheter and collection bag that can be provided without additional packaging. For example, the urinary catheter is contained in the collection bag and the collection bag can be folded to form a package for the catheter and any additional components or printed information.

In an embodiment, the present invention relates to a catheter set including a collection bag and a urinary catheter. The catheter is inside the collection bag. The collection bag is configured as the package for the set. The set is not in packaging beyond the collection bag. The set can also include other items used for or while handling or inserting a urinary catheter. Such items can include an introducer tip, a wipe, a lubricant, a pad, a glove, or a plurality thereof. In the present set, these items are packaged by the collection bag. For example, the collection bag can be folded or wrapped around these items. The collection bag can be coupled to itself when it is folded or wrapped around these items to form (e.g., close or seal) the package.

The present invention includes a urine collection bag configured as a package for the catheter set. As a package, the urine collection bag is folded or wrapped to contain components of the set. The collection bag can be secured around the components it packages. For example, adhesive can couple one portion of the bag to another portion of the bag. For example, a seal (e.g., paper backed with adhesive) can be used to couple one portion of the bag to another portion of the bag. The bag can be secured to itself with sufficient strength to maintain integrity of the package in a case of these packages or when carried by a user or health care professional. The components contained within the folded or wrapped collection bag are not in the interior of the bag. These components may contact the exterior surface of the collection bag, but they do not contact its interior surface.

The interior of the bag is the space in which urine collects when the bag is in use. The catheter and an optional fluid (e.g., water) packet and optional gripping device are in the interior of the bag. In an embodiment, other components are not in the interior of the bag. After the bag is opened, the end of the catheter that is first introduced into the subject may protrude slightly from the bag when the bag is configured as the package and when the catheter is inside the bag. The end of the catheter that is first introduced into the subject may be inside an introducer tip when the bag is configured as the package. In an embodiment, the entire catheter (including the end that is first introduced into the subject) is inside the bag, in the space in which urine collects, when the bag is configured as the package with the end that is first introduced into the subject positioned near the opening of the bag. The flow of urine in the catheter enters the bag through the opening of the bag.

The collection bag can include one or more optional flaps to aid in containing the other components of the set. A flap does not include an interior space that can contain urine. Of course, the bag can have a shape that provides regions that define a portion of the interior of the bag but that resemble a flap. As used herein, however, the term flap refers to a portion of the bag that does not define an interior space.

Although the present catheter set does not require or include packaging beyond the bag, individual components of the set may be within their own package. For example, a set including a hydrophilic catheter can include water in a packet. At the time of use of the catheter, the packet is breached to release the water, which increases the slipperiness of the hydrophilic catheter. The packet is a container for the water. The bag as a package contains the water packet. Similarly, a lubricant or a (e.g., antibacterial) wipe can be in a packet. Such a packet is not a package beyond the bag. The bag as package contains the packet of lubricant or wipe. Similarly, a cover for the introducer tip or for the opening in the bag is not packaging beyond the bag.

Components of the set can include, for example, an introducer tip, a wipe, a lubricant, a pad, a glove, or a plurality thereof. Suitable wipes include antibacterial wipes that the person inserting the catheter can use to wipe around the subject's urethra. The wipe can be used on the hands of the person inserting the catheter. The set can include one or more wipes. Each wipe can be in a packet. Suitable pads include an absorbent pad for absorbing any leaked urine. Suitable gloves include plastic or latex gloves, e.g. examination gloves. The gloves can be provided as a pair of gloves. The gloves can be used to prevent contact of the skin of the person inserting the catheter with the catheter and/or tissues near the subject's urethra.

The catheter can be any of a variety of urinary catheters. For example, the catheter can be a hydrophilic catheter. The catheter can be made of silicone rubber and can be latex-free. The entire set can be latex free.

In an embodiment, the present urinary catheter set includes a collection bag and a urinary catheter. The catheter can be inside the collection bag. The collection bag can be configured as a package for the set. And the collection bag is the only package for the set.

The set can also include a fluid packet. The fluid packet can be inside the collection bag. The set can also include an introducer tip, a wipe, a lubricant, a pad, a glove, or a plurality thereof. The introducer tip, wipe, lubricant, pad, glove, or plurality thereof can be packaged by the collection bag. For example, the collection bag can be folded around the introducer tip, wipe, lubricant, pad, glove, or plurality thereof and coupled to itself to form the package for the set. In an embodiment, the set includes the introducer tip, the wipe, the lubricant, the pad, and the glove. In an embodiment, the introducer tip includes an introducer tip cover releaseably coupled to the introducer tip. In an embodiment, the set does not include additional lubricant.

In an embodiment, the present invention includes a case of urinary catheter sets. The case includes a container and a plurality of catheter sets. Each catheter set in the case includes a collection bag and a urinary catheter. The catheter is inside the collection bag, the collection bag is configured as a package for the set, and the set includes no additional packaging. The container contains the plurality of catheter sets. The container can be, for example, a box, a tray, or a bag.

In an embodiment, the urinary catheter can be contained in the collection bag and the collection bag can be folded to form a package for the catheter and any additional components or printed information.

### Illustrated Embodiments

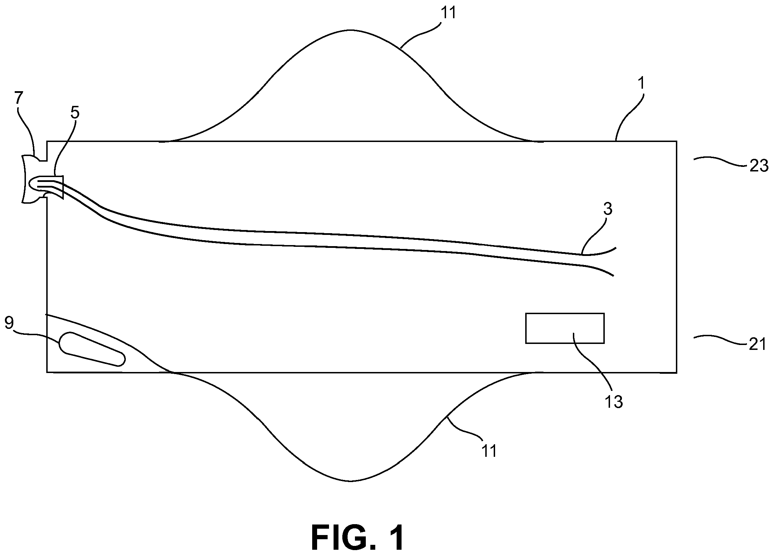

Figure 1 schematically illustrates an embodiment of the present collection bag and urinary catheter. Bag 1 is shown containing straight catheter 3. Straight catheter 3 is equipped with introducer tip 5 proximal opening 7 of bag 1. Bag 1 includes optional grip 9 (an embodiment of the handle) and optional extensions 11 (which are embodiments of the flap). Also shown is sachet 13, which is an embodiment of the fluid packet.

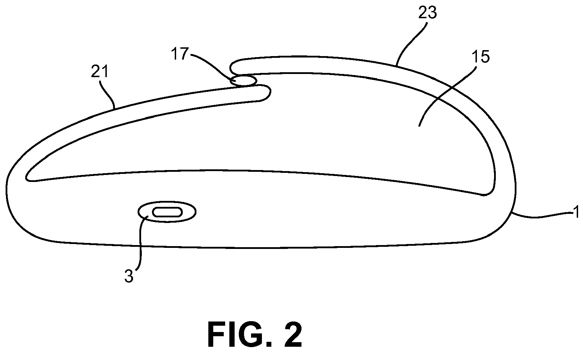

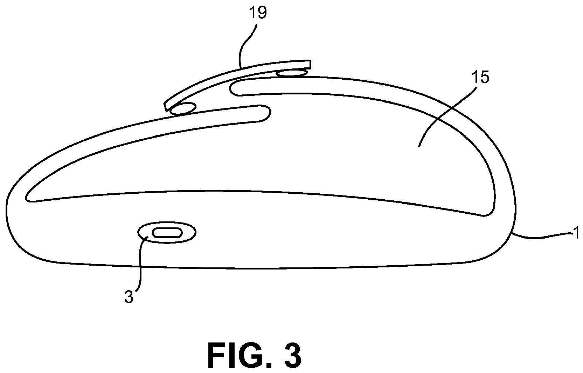

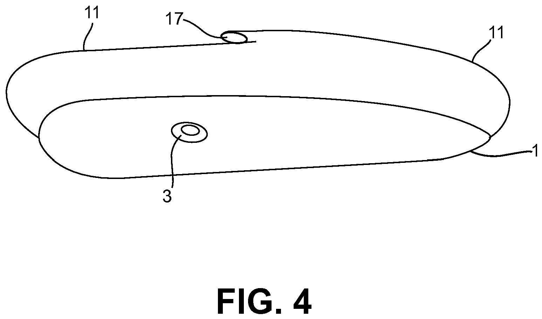

Figure 2 schematically illustrates a cross sectional view of an embodiment of bag 1 in a configuration in which it has been wrapped or folded to form cavity 15. Cavity 15 can contain additional items such as an introducer tip, a wipe, a lubricant, a pad, a glove, or a plurality thereof. These additional items are not shown in the figures. Bag 1 can be secured in the configuration having cavity 15 by, for example, adhesive 17 or paper seal 19 (Figure 3). In the embodiment shown in Figures 2 and 3, side portions 21 and 23 of bag 1 are folded or wrapped to form cavity 19. Figure 4 schematically illustrates an embodiment in which two extensions 11 are wrapped or folded to form cavity 19.

It should be noted that, as used in this specification and the appended claims, the singular forms "a," "an," and "the" include plural referents unless the content clearly dictates otherwise. Thus, for example, reference to a composition containing "a compound" includes a mixture of two or more compounds. It should also be noted that the term "or" is generally employed in its sense including "and/or" unless the content clearly dictates otherwise.

It should also be noted that, as used in this specification and the appended claims, the term "configured" describes a system, apparatus, or other structure that is constructed or configured to perform a particular task or adopt a particular configuration. The term "configured" can be used interchangeably with other similar phrases such as arranged and configured, constructed and arranged, adapted and configured, adapted, constructed, manufactured and arranged, and the like.

All publications and patent applications in this specification are indicative of the level of ordinary skill in the art to which this invention pertains.

The invention has been described with reference to various specific and preferred embodiments and techniques. However, it should be understood that many variations and modifications may be made while remaining within the scope of the invention.

## Claims

1. A urinary catheter set, comprising:
a collection bag (1) having an interior which is the space in which urine collects when the collection bag is in use; and
a urinary catheter (3);
the entire catheter being inside the space in which urine collects, the collection bag being configured as a package for the set, and the collection bag is the only package for the set.

2. The catheter set of claim 1, further comprising a fluid packet, the fluid packet being inside the collection bag.

3. The catheter set of claim 1, further comprising an introducer tip (5), a wipe, a lubricant, a pad, a glove, or a plurality thereof, the introducer tip, wipe, lubricant, pad, glove, or plurality thereof being packaged by the collection bag.

4. The catheter set of claim 3, wherein the collection bag is folded around the introducer tip, wipe, lubricant, pad, glove, or plurality thereof and coupled to itself to form the package for the set.

5. The catheter set of claim 3, comprising the introducer tip, the wipe, the lubricant, the pad, and the glove.

6. The catheter set of claim 3, comprising the introducer tip, wherein the introducer tip comprises an introducer tip cover releaseably coupled to the introducer tip.

7. A case of urinary catheter sets comprising:
a container and a plurality of urinary catheter sets according to any preceding claim, wherein the container contains the plurality of catheter sets.

8. The case of catheter sets of claim 7, wherein the container is a box, a tray, or a bag.

9. The catheter set of any of claims 1 to 6, wherein the collection bag further comprises a first and second extension, which do not define an interior space of the collection bag, the first and second extension configured to be folded to form a cavity.

## Patentansprüche

1. Harnkathetersatz, umfassend:
einen Auffangbeutel (1), einen Innenraum aufweisend, der der Raum ist, in dem Urin aufgefangen wird, wenn der Auffangbeutel verwendet wird; und
einen Harnkatheter (3);
wobei sich der gesamte Katheter in dem Raum befindet, in dem der Urin aufgefangen wird, wobei der Auffangbeutel als eine Verpackung für den Satz konfiguriert ist und der Auffangbeutel die einzige Verpackung für den Satz ist.

2. Kathetersatz nach Anspruch 1, weiter umfassend ein Fluidpaket, wobei sich das Fluidpaket innerhalb des Auffangbeutels befindet.

3. Kathetersatz nach Anspruch 1, weiter umfassend eine Einführspitze (5), ein Wischtuch, ein Gleitmittel, ein Kissen, einen Handschuh oder eine Vielzahl davon, wobei die Einführspitze, das Wischtuch, das Gleitmittel, das Kissen, der Handschuh oder die Vielzahl davon von dem Auffangbeutel verpackt sind.

4. Kathetersatz nach Anspruch 3, wobei der Auffangbeutel um die Einführspitze, das Wischtuch, das Gleitmittel, das Kissen, den Handschuh oder die Vielzahl davon gefaltet und mit sich selbst gekoppelt ist, um die Verpackung für den Satz zu bilden.

5. Kathetersatz nach Anspruch 3, umfassend die Einführspitze, das Wischtuch, das Gleitmittel, das Kissen und den Handschuh.

6. Kathetersatz nach Anspruch 3, umfassend die Einführspitze, wobei die Einführspitze eine Einführspitzenabdeckung umfasst, die lösbar mit der Einführspitze gekoppelt ist.

7. Gehäuse für Harnkathetersätze, umfassend:
einen Behälter und eine Vielzahl von Harnkathetersätzen nach einem der vorstehenden Ansprüche, wobei der Behälter die Vielzahl von Kathetersätzen enthält.

8. Gehäuse für Kathetersätze nach Anspruch 7, wobei der Behälter eine Schachtel, eine Schale oder ein Beutel ist.

9. Kathetersatz nach einem der Ansprüche 1 bis 6, wobei der Auffangbeutel weiter eine erste und eine zweite Verlängerung umfasst, die keinen Innenraum des Auffangbeutels definieren, wobei die erste und die zweite Verlängerung konfiguriert sind, um gefaltet zu werden, um einen Hohlraum zu bilden.

## Revendications

1. Ensemble de cathéter urinaire, comprenant :
une poche de recueil (1) ayant un intérieur qui est l'espace dans lequel l'urine est recueillie lorsque la poche de recueil est utilisée ; et
un cathéter urinaire (3) ;
le cathéter entier étant à l'intérieur de l'espace dans lequel l'urine est recueillie, la poche de recueil étant configurée comme un emballage pour l'ensemble, et la poche de recueil est le seul emballage pour l'ensemble.

2. Ensemble de cathéter selon la revendication 1, comprenant en outre un sachet de fluide, le sachet de fluide étant à l'intérieur de la poche de recueil.

3. Ensemble de cathéter selon la revendication 1, comprenant en outre un embout d'introduction (5), une lingette, un lubrifiant, un tampon, un gant, ou une pluralité de ceux-ci, l'embout d'introduction, la lingette, le lubrifiant, le tampon, le gant, ou une pluralité de ceux-ci étant emballés par la poche de recueil.

4. Ensemble de cathéter selon la revendication 3, dans lequel la poche de recueil est repliée autour de l'embout d'introduction, de la lingette, du lubrifiant, du tampon, du gant, ou d'une pluralité de ceux-ci et couplée à elle-même de façon à former l'emballage pour l'ensemble.

5. Ensemble de cathéter selon la revendication 3, comprenant l'embout d'introduction, la lingette, le lubrifiant, le tampon et le gant.

6. Ensemble de cathéter selon la revendication 3, comprenant l'embout d'introduction, dans lequel l'embout d'introduction comprend un capuchon d'embout d'introduction couplé de façon amovible à l'embout d'introduction.

7. Étui d'ensembles de cathéters urinaires comprenant :
un contenant et une pluralité d'ensembles de cathéters urinaires selon une quelconque revendication précédente, dans lequel le contenant contient la pluralité d'ensembles de cathéters.

8. Étui d'ensembles de cathéters selon la revendication 7, dans lequel le contenant est une boîte, un plateau, ou une poche.

9. Ensemble de cathéter selon l'une quelconque des revendications 1 à 6, dans lequel la poche de recueil comprend en outre un premier et un second prolongement qui ne définissent pas un espace intérieur de la poche de recueil, les premier et second prolongements étant configurés pour être repliés de façon à former une cavité.
